# EUROPEAN PATENT APPLICATION

(11) **EP 4 549 557 A1**
(43) Date of publication of application: **07.05.2025**
(21) Application number: 22949543.7
(22) Date of filing: 08.07.2022
(51) Int. Cl.: C12N 7/00, C07K 14/705, A61K 35/768, A61P 35/00

(54) **ONCOLYTIC VIRUS SIMULTANEOUSLY EXPRESSING CD55 AND CD59**

(30) Priority: 29.06.2022 KR 20220080056
(71) Applicant: SillaJen, Inc., Seoul 04525 (KR)
(72) Inventor: OH, Keunhee, Seongnam-si Gyeonggi-do 13615 (KR); LEE, Namhee, Seongnam-si Gyeonggi-do 13555 (KR); LEE, Songyi, Seoul 04722 (KR)
(74) Representative: J A Kemp LLP
(86) International application number: PCT/KR2022/009910
(87) International publication number: WO 2024/005250

(57) **Abstract**

The present invention relates to an oncolytic virus co-expressing the complement regulatory proteins CD55 and CD59.

The oncolytic virus of the present invention maintains efficacy even upon intravenous injection, and thus, it may be applied to the therapy for various solid carcinomas and metastatic cancers as well as superficial solid cancers. In addition, the oncolytic virus of the present invention acquires resistance to the attack of human complement system by expressing a complement regulatory protein on the surface of the virus, and thus, it is stable in blood, and it maintains stable oncolytic activity upon intravenous injection, and thus, it may reduce the dose of the virus to minimize adverse effects of anticancer agents. Therefore, the oncolytic virus of the present invention may be advantageously used for prevention or treatment of cancer.

## Description

### [Technical Field]

The present invention relates to an oncolytic virus co-expressing the complement regulatory proteins CD55 and CD59.

### [Background Art]

Cancer is a leading cause of death worldwide, and with its enormous personal and societal burden, the importance of developing innovative anticancer treatments has gradually increased. Accordingly, various approaches based on advances in molecular biology have been attempted.

Oncolytic virus, an anticancer treatment that has recently been in the spotlight, is replicable and infectious virus that is used to treat cancer by inserting specific genes into a wild-type or attenuated virus. These genetically engineered oncolytic viruses spread through tissues with cell lysis after viral replication, and their spread occurs selectively in cancer cells and blood vessels surrounding the cancer cells. A representative oncolytic virus is lmlygic (talimogene laherparepvec) from Amgen in the United States, which has been approved by the U.S. Food and Drug Administration (FDA) as a treatment for melanoma in October 2015.

Meanwhile, intravenous injection of medicines is a convenient method to rapidly deliver drugs systemically when oral administration is not possible, and it is simple to administer, making it one of the most preferred routes of administration. However, since oncolytic virus preparations are quickly eliminated by the body's immune system upon intravenous injection, there is a limitation that their efficacy decrease when administered through intravenous injection. Therefore, in order to increase the probability of the virus reaching the tumor, oncolytic virus preparations are commonly injected directly into the target tumor rather than intravenously.

Intratumoral injection may generally be effectively applied in superficial cancers that are easily accessible, such as melanoma, breast cancer, and head and neck cancer, but has the disadvantage that the administration of an effective dose is dependent on the technical ability of the physicians performing the procedure in cancers such as deep solid tumor. In addition, intratumoral injection is very invasive procedure, and thus is not easy to use as a repeat treatment method compared to intravenous injection.

Despite the advantages of intravenous injection, the reason why oncolytic viruses are difficult to administer in the form of intravenous injection is that foreign substances (i.e. viruses) administered into the blood are gradually removed by the body's defense mechanism, thereby reducing the activity of the oncolytic virus. In other words, when a virus enters the body through the blood vessels, the human body recognizes it as a threat and activates innate immune system to neutralize and eliminate it.

When the human body is exposed to foreign invading bacteria or viruses, the complement system, which is first line of defense mechanism in the innate immune system, acts first. The complement is a protein present in the blood and surrounds a surface of foreign microorganisms to facilitate phagocytosis by macrophages or neutrophils. Specifically, when the virus enters the bloodstream, the proteins on a viral surface first bind to the complement, causing them to aggregate and activate the complement system. The activated complements surround the viral surface to facilitate phagocytosis by phagocytes (opsonization), and create holes in the viral surface to lyse the virus. The viral debris thus lysed is completely removed through phagocytosis by macrophages.

In order to protect surrounding normal cells from damage caused by excessive activation of the complement system, the human body expresses complement regulatory proteins such as CD55, CD46, and CD59 on a cell surface to control excessive complement activity.

Some (about 5 to 20%) of oncolytic viruses that proliferated in host cells are characterized by the form of "extracellular enveloped viruses (EEVs)" produced in a manner that is surrounded by a cell membrane of infected cells. These EEVs are known to evade complement activation, resulting in a long survival period in the blood. The mechanism has been shown to be due to complement regulatory proteins, such as CD55, CD46 and CD59, which are expressed on the cell membrane of the host cell from which the virus is enveloped. For example, most of the produced oncolytic vaccinia viruses are in the form of "intracellular mature virus (IMVs)," which do not express complement regulatory proteins on the surface, leading to a significant reduction in activity by complement. As a result, there is a need for research and development to improve the oncolytic activity upon intravenous injection of oncolytic viruses.

### [Disclosure]

### [Technical Problem]

An object of the present disclosure is to provide an oncolytic virus co-expressing CD55 and CD59.

Another object of the present disclosure is to provide a pharmaceutical composition for preventing or treating cancer, comprising the oncolytic virus described above as an active ingredient.

Another object of the present disclosure is to provide a gene construct for insertion into an oncolytic virus, comprising all or part of a gene encoding CD55 and CD59 and operably linked to a promoter for expression.

Another object of the present disclosure is to provide an anticancer adjuvant comprising the oncolytic virus described above as an active ingredient.

Another object of the present disclosure is to provide a method of preventing or treating cancer, comprising administering to a subject a composition comprising the oncolytic virus described above as an active ingredient.

Another object of the present disclosure is to provide a use of the oncolytic virus described above or a composition comprising the oncolytic virus, for the prevention or treatment of cancer.

Another object of the present disclosure is to provide a use of the oncolytic virus described above or a composition comprising the oncolytic virus, for the manufacture of a medicament for the prevention or treatment of cancer.

The technical objectives to be achieved according to the technical idea of the present disclosure disclosed in the specification are not limited to the objectives for solving the problems mentioned above, and other objectives not mentioned may also be clearly understood by those skilled in the art from the following description.

### [Technical Solution]

This will be specifically described as follows. Meanwhile, each of the descriptions and embodiments disclosed in the present application may also be applied to each other descriptions and embodiments. That is, all combinations of various elements disclosed in the present application fall within the scope of the present application. In addition, the scope of the present application cannot be considered limited by the specific description set forth below.

As one aspect of achieving the above-mentioned objectives of the present disclosure, the present disclosure provides an oncolytic virus co-expressing CD55 and CD59.

In particular, in the present disclosure, as an example, a transmembrane domain in a membrane protein of the oncolytic virus itself was linked to a CD55 gene, and a membrane protein of the oncolytic virus itself was linked to the CD59 gene, enabling the CD55 and CD59 proteins to be expressed on the surface of an "intra-membrane mature virus" (IMV). Through this approach, the virus is made resistant to the human complement system, ensuring stable oncolytic activity upon intravenous injection, while also minimizing therapeutic side effects by reducing the dose of the oncolytic virus.

As used herein, the term "oncolytic vaccinia virus" may be referred to as an "oncolytic virus," which includes a "recombinant virus" engineered by the deletion of all or part of an endogenous gene, or the introduction of a foreign gene. Such an oncolytic virus may be a vaccinia virus, an adenovirus, a herpes simplex virus, a retrovirus, a reovirus, a Newcastle disease virus, a Coxsackie virus, an enterovirus, or a herpesvirus.

In the present disclosure, the expression of a thymidine kinase gene in the oncolytic virus may be suppressed.

As used herein, the term "thymidine kinase (TK)" refers to an enzyme involved in the biosynthesis of nucleotides. The thymidine kinase encoded by the TK gene can bind phosphate at the gamma (γ) position of ATP to thymidine to produce nucleotides that make up viral DNA. The TK may be, but is not limited to, a sequence such as GenBank: AAR17937.1 or AY313847.1. Specifically, the TK or its gene may be comprised of an amino acid sequence of GenBank: AAR17937.1 or a nucleotide sequence of GenBank: AY313847.1, but is not limited thereto. In addition, the TK or its gene may have a homology of about 90%, 91%, 92%, 93%, 94%, 95%, 96%, 97%, 98%, or 99% or more to the amino acid sequence of GenBank: AAR17937.1 or the nucleotide sequence of GenBank: AY313847.1.

As used herein, the term "complement regulatory protein" refers to a protein that precisely regulates a complement activation pathway *in vivo.* It is broadly divided into a serotype (soluble) regulatory protein group and a membrane-bound regulatory protein group. The serotype includes C4b-binding protein, H factor, SGP120, and properdin (P), etc. The membrane type includes CRI (CD35), CR2 (CD21), CR3 (CD11b/CD18), CR4 (CD11c/CD18), DAF (CD55), membrane cofactor protein (MCP, CD46), and CD59. Of these, only P acts to enhance complement activity, while the others act to attenuate complement activity. The complement regulatory protein contained in the recombinant vaccinia virus of the present disclosure may be, but is not limited to, CD35, CD21, CD18, CD55, CD46, or CD59, and specifically CD55 and/or CD59.

Specifically, the gene of the CD55 may be comprised of a nucleotide sequence of Genbank: NM_000574.3 or may be part of the sequence. The gene of the CD55 may have a homology of about 90%, 91%, 92%, 93%, 94%, 95%, 96%, 97%, 98%, or 99% or more to the nucleotide sequence of NCBI Reference Sequence: NM_000574.3. More specifically, the CD55 may be comprised of an amino acid sequence of SEQ ID NO: 1 or a nucleotide sequence of SEQ ID NO: 2.

Specifically, the gene of the CD59 may be comprised of a nucleotide sequence of Genbank: NM_203331.3, or may be part of the nucleotide sequence. The gene of the CD59 may have a homology of about 90%, 91%, 92%, 93%, 94%, 95%, 96%, 97%, 98%, or 99% or more to the nucleotide sequence of NCBI Reference Sequence: NM_203331.3. More specifically, the CD59 may be comprised of an amino acid sequence of SEQ ID NO: 3 or a nucleotide sequence of SEQ ID NO: 4.

As used herein, the term "transmembrane domain" is also called a membrane-penetrating region or a membrane-transversing region and refers to a region that penetrates and crosses a lipid bilayer of a membrane protein.

An oncolytic virus according to the present disclosure may further comprise a gene encoding an oncolytic virus membrane protein or a transmembrane domain thereof.

In the present disclosure, as an example, the CD55 and/or CD59 may be expressed separately and independently from the gene encoding the transmembrane domain, and may be engineered to be expressed in a linked fusion.

In the present disclosure, as an example, the gene encoding the oncolytic virus membrane protein may be linked to the gene encoding CD59 via a linker. More specifically, the linker may be (G4S)₃, comprised of an amino acid sequence of SEQ ID NO: 7 or a nucleotide sequence of SEQ ID NO: 8, but is not limited thereto.

In the present disclosure, the membrane protein of a vaccinia virus may be H3L, A27L, D8L, A16L, F9L, G9R, L1R, A9L, A13L, A21L, A28L, E10R, G3L, H2R, I2L, J5L, L5R, or O3L, and may comprise all or part of the membrane protein sequences, as shown in Table 1 below for example.

**[Table 1]**

| Membrane protein | Section | Sequence | SEQ ID NO: |
|---|---|---|---|
| H3L | Amino acid | LFDINVIGLIVILFIMFMLIFNVKSKLLWFL TGTFVTAFI | 9 |
| | DNA | ttgtttgatattaatgttataggtttgattgtaattttgtttattatgtttatg ctcatctttaacgttaaatctaagctgttatggttccttacaggaacat tcgttaccgcatttatc | 10 |
| A27L | Amino acid | MDGTLFPGDDDLAIPATEFFSTKAAKKPE AKREAIVKADEDDNEETLKQRLTNLEKKI TNVTTKFEQIEKCCKRNDEVLFRLENHAE TLRAAMIS LAKKIDVQTGRRPYE | 11 |
| | DNA | atggacggaactcttttccccggagatgacgatcttgcaattccag caactgaatttttttctacaaaggctgctaaaaagccagaggctaaa cgcgaagcaattgttaaagccg atgaagacgacaatgaggaaactctcaaacaacggctaactaattt ggaaaaaaagattactaatgtaacaacaaagtttgaacaaatagaa aagtgttgtaaacgcaacga tgaagttctatttaggttggaaaatcacgctgaaactctaagagcgg ctatgatatctctggctaaaaagattgatgttcagactggacggcgt ccatatgag | 12 |
| D8L | Amino acid | FAIIAIVFVFILTAILFFMSRRYSREKQN | 13 |
| | DNA | ttcgcaattattgccatagtattcgtgtttatacttaccgctattctctttt ttatgagtcgacgatattcgcgagaaaaacaaaac | 14 |
| A16L | Amino acid | LGAAITLVVISVIFYFISIYSRPKIKTNDINV RRR | 15 |
| | DNA | ttgggcgcggccataacattggttgtaatatctgttattttctattttata tctatttattcgcgtactaaaattaaaacaaatgatataaatgttcgta gacga | 16 |
| F9L | Amino acid | PWFLVGVAIILVIFTVAICSIRRNLALKYRY GTFLYV | 17 |
| | DNA | ccgtggtttctagtgggtgtagcaattattctagttatttttactgtagc tatttgttctattagacgaaatctggctcttaaatacagatacggaac gtttttatacgtt | 18 |
| G9R | Amino acid | LKLHLISLLSLLVIWILIVAI | 19 |
| | DNA | ctaaaattgcatttgatcagtttattatctctcttggtaatatggatacta attgtagctatt | 20 |
| L1R | Amino acid | VQFYMIVIGVIILAALFMYYAKRMLFTSTN DKIKLILANKENVHWTTYMDTFFRTSPMV IATTDMQN | 21 |
| | DNA | gttcagttttatatgattgttatcggtgttataatattggcagcgttgttt atgtactatgccaagcgtatgttgttcacatccaccaatgataaaatc aaacttattttagccaataaggaaaacgtccattggactacttacatg gacacattctttagaacttctccgatggttattgctaccacggatatg caaaac | 22 |
| A9L | Amino acid | FVVVRAIASMIMYLVLGIALL | 23 |
| | DNA | ttcgtcgttgttagagccattgcgagcatgataatgtatttagtattag gtatagcattgctg | 24 |
| A13L | Amino acid | MIGILLLIGICVAVTVAILYA | 25 |
| | DNA | atgattggtattcttttgttgatcggtatttgcgtagcagttaccgtcg ccatcctatatgcg | 26 |
| A21L | Amino acid | MITLFLILCYFILIFNIIVPA | 27 |
| | DNA | atgataactttatttttaatcctatgttatttcattcttatttttaatattatag tacctgca | 28 |
| A28L | Amino acid | MNSLSIFFIVVATAAVCLLFI | 29 |
| | DNA | atgaactctctatcaattttttttattgtggtagcgacggctgcggtgt gtttactttttatc | 30 |
| E10R | Amino acid | AVWTIIFIVLSQAGLDG | 31 |
| | DNA | gccgtatggaccattatttttatagtactttcgcaagcgggtttagac ggc | 32 |
| G3L | Amino acid | MASLLYLILFLLFVCISYYFT | 33 |
| | DNA | atggcatctttattatatcttattttatttttgttattcgtatgtatttcttatt attttaca | 34 |
| H2R | Amino acid | TSTLIFFVIILAISALLLWFQ | 35 |
| | DNA | acatcaacacttatattctttgttattatattggcaattagtgcgctatta ctctggtttcag | 36 |
| I2L | Amino acid | YWLIIIFFIVLILLLLIYLYL | 37 |
| | DNA | tattggttaattattattttttttatagttcttattctactactattgatatattt gtattta | 38 |
| J5L | Amino acid | IIDIKWLPIGLLALAILILAF | 39 |
| | DNA | attatagatatcaaatggcttccgattggattactagcgttagctatttt aatattagcattt | 40 |
| L5R | Amino acid | IVLFEVFVVFILIYVFFRSEL | 41 |
| | DNA | atagttttatttgaagtattcgttgtattcattctaatatatgtattttttag atctgaatta | 42 |
| O3L | Amino acid | LVVIMFFIAFAFCSWLSYSYL | 43 |
| | DNA | ctcgtcgtaattatgttttttatagcgtttgccttctgtagttggctatca tatagctatctg | 44 |

Here, CD55 and/or CD59 and the oncolytic viral protein or transmembrane domain thereof may be designed to include all or part of the gene through a genetic engineering process. Specifically, among the oncolytic virus, the transmembrane domain of the vaccinia virus membrane protein may be comprised of, but is not limited to, a nucleotide sequence of SEQ ID NO: 9, SEQ ID NO: 13, SEQ ID NO: 15, SEQ ID NO: 17, SEQ ID NO: 19, or SEQ ID NO: 21, or a nucleotide sequence of SEQ ID NO: 10, SEQ ID NO: 14, SEQ ID NO: 16, SEQ ID NO: 18, SEQ ID NO: 20, or SEQ ID NO: 22.

Gene expression inhibition or gene inactivation according to the present invention refers to the deletion of part or all of the gene, or the insertion of a foreign gene into the gene, such that the gene is not expressed or only a portion of the gene is expressed, resulting in the absence of activity of the protein encoded by the gene. The method of deleting the gene or inserting the foreign gene may be performed by methods well known in the art. For example, it may be performed by a method of inserting a foreign gene disclosed in Molecular Cloning, A Laboratory Manual, Second Edition, by J. Sambrook, E. F. Fritsch and T. Maniatis (2003), Cold Spring Harbor Laboratory Press, Virology Methods Manual, edited by Brian W J Mahy and Hiliar O Kangro (1996) Academic Press and Expression of genes by Vaccinia virus vectors. This can be done by inserting a foreign gene as disclosed in Current Protocols in Molecular Biology, published by John Wiley and Son (1998), Chapter 16. Specifically, the suppression of the expression of the thymidine kinase gene may be caused by insertion of a foreign gene into part or all of a J2R region of the thymidine kinase. More specifically, in one embodiment of the present disclosure, the foreign gene was inserted using a T-Blunt^{™} PCR Cloning Kit (Solgent, Korea Cat No. SOT01-K020). The CD55 and/or CD59 contained in the oncolytic virus of the present disclosure may be expressed under the control of, but are not limited to, a late-early VACV p7.5 promoter, a vaccinia synthetic early-late promoter (pSEL), a vaccinia synthetic late promoter (pSL), a vaccinia modified H5 (mH5) promoter, a vaccinia short synthetic early-late pS promoter, a pLEO160 promoter, a pLEO38 promoter, a plate promoter, a pC11R promoter, a pF11L promoter, a psFJ1-10 synthetic early promoter, a pHyb synthetic early promoter, any natural vaccinia early promoter, or a late-early optimized (LEO) promoter, respectively. As an example, the complement regulatory protein CD55 or CD59 may be expressed under the control of the pLEO160 or pLEO38 promoter. More specifically, the CD55 may be expressed under the control of the pLEO160 promoter of SEQ ID NO: 5, and the CD59 may be expressed under the control of the pLEO38 of SEQ ID NO: 6.

The oncolytic virus of the present disclosure may further comprise genes that can enhance cancer treatment efficacy. These genes are not limited, but may include, for example, anticancer therapeutic genes, various immunomodulatory factors, and structural decomposition factors such as fibers in tumor, etc. The oncolytic virus may further include, for example, but is not limited to, IL-1, IL-2, IL-3, IL-4, IL-5, IL-6, IL-7, IL-8, IL-9, IL-10, IL-12, IL-13, IL-14, IL-15, IL-17, IL-18, IL-21, IL-23, IL-24, interferon-α, interferon-β, interferon-γ, CCL3, CCL5, CXCR4, CXCL9, CXCL10, CXCL11, interleukin superagonist (IL-2 superagonist, IL-15 superagonist), TGF-β blockade, TLR-2 agonist, TLR-3 agonist, TLR-7 agonist, STAT-3 inhibitor, PTENα, p53, p63, p73, adenosine deaminase-2, cancer-specific antigen, cancer-associated antigen, hyaluronidase, collagenase, protease, etc.

In the present disclosure, the oncolytic virus includes a vaccinia virus, an adenovirus, a herpes simplex virus, a retrovirus, a reovirus, a Newcastle disease virus, a Coxsackie virus, an enterovirus, a herpesvirus, etc. The oncolytic virus may be, for example, but is not limited to, Western Reserve (WR), New York Vaccinia Virus (NYVAC), Wyeth, LC16m8, Lister, Copenhagen, Tian Tan, USSR, TashKent, Evans, International Health Division-J (IHD-J), or International Health Division-White (IHD-W) strain.

In another aspect to achieve the above objectives, the present disclosure provides a pharmaceutical composition for preventing or treating cancer comprising the oncolytic virus as an active ingredient.

As used therein, the term "active ingredient" refers to an ingredient capable of exhibiting the intended activity alone or in combination with a carrier that is inactive in itself.

As used herein, the term "cancer (or tumor)" includes all cancers (or tumors), regardless of whether they are primary cancers or metastatic cancers. In the present disclosure, the cancer may be solid cancer or haematological cancer. The solid cancer may be any one selected from the group consisting of, but is not limited to, lung cancer, colorectal cancer, prostate cancer, thyroid cancer, breast cancer, brain cancer, head and neck cancer, esophageal cancer, skin cancer, thymic cancer, stomach cancer, colon cancer, liver cancer, ovarian cancer, uterine cancer, bladder cancer, rectal cancer, gallbladder cancer, bile duct cancer, pancreatic cancer, kidney cancer, osteosarcoma, sarcoma, chondrosarcoma, and combinations thereof. The haematological cancer may be any one selected from the group consisting of, but not limited to, lymphoma, leukaemia, multiple myeloma, and combinations thereof.

As used herein, the term "metastatic cancer" refers to cancer that occurs when cancer cells leave the primary organ, spread to other organs, and proliferate. The metastatic cancer may include, but is not limited to, both the growth of cancerous tissue from the primary cancer that directly invades surrounding organs, and remote metastasis to another organ at a distance along a blood vessel or lymphatic vessel.

As used herein, the term "prevention" means any act of suppressing or delaying the development of cancer through the administration of a pharmaceutical composition of the present disclosure. As used therein, the term "treatment" means any act of ameliorating, alleviating, or beneficially altering cancer by administering a pharmaceutical composition of the present disclosure.

The specific dosage of a pharmaceutical composition comprising an oncolytic virus of the present disclosure may be selected in various ways by those skilled in the art depending on factors such as a formulation method, the patient's condition and weight, the patient's sex, age, disease severity, drug form, administration route and duration, excretion rate, response sensitivity, etc., and the dosage and frequency are not intended to limit the scope of the present disclosure in any way. Specifically, the pharmaceutical composition of the present disclosure may comprise, but is not limited to, from 10⁵ to about 10¹³ plaque forming units (pfu) of an oncolytic virus.

As used herein, the term "pharmaceutical composition" refers to a composition prepared for the purpose of preventing or treating a disease, each of which may be formulated and used in various forms according to methods known in the art. For example, it may be formulated as an oral formulation such as powders, granules, tablets, capsules, suspensions, emulsions, syrups, etc., depending on the administration route, and may be formulated and used in the form of an external preparation and a sterile injection solution. Specifically, the administration route may be any appropriate route, including a local route, an oral route, an intravenous route, an intramuscular route, and direct absorption through mucosal tissue, or a combination of two or more routes. An example of a combination of two or more routes of administration is where drugs of two or more formulations are combined according to the administration route, such as administering one drug initially as the primary route and administering another drug locally as the secondary route.

The pharmaceutical compositions of the present disclosure may be for intratumoral, intravascular, intramuscular, or intraperitoneal administration, and as an example, may be for intravenous or intraarterial administration.

The pharmaceutical composition of the present disclosure may be prepared in the form of a pharmaceutical composition for the treatment or prevention of cancer, further comprising any appropriate carrier, excipient, or diluent conventionally used in the preparation of pharmaceutical compositions, wherein the carrier may comprise a non-naturally occurring carrier. Specifically, the pharmaceutical composition may be formulated and used in the form of oral formulations, such as powders, granules, tablets, capsules, suspensions, emulsions, syrups, aerosols, external preparations, suppositories, and sterile injection solutions, respectively, according to conventional methods. In the present disclosure, examples of carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. When formulating the preparation, diluents or excipients such as commonly used fillers, bulking agents, binders, wetting agents, disintegrants, and surfactants are used. Solid preparations for oral administration include tablets, pills, powders, granules, capsules, etc., and these solid preparations are prepared by mixing at least one excipient, for example, starch, calcium carbonate, sucrose or lactose, gelatin, etc. Further, in addition to simple excipients, lubricants such as magnesium stearate and talc are also used. Liquid preparations for oral administration include suspensions, solutions, emulsions, syrups, etc., and may include a variety of excipients, for example, wetting agents, sweeteners, flavoring agents, preservatives, etc., in addition to simple diluents commonly used, such as water and liquid paraffin. Preparations for parenteral administration include sterile aqueous solutions, non-aqueous solutions, suspensions, emulsions, lyophilized preparations, and suppositories. Non-aqueous solvents and suspensions may include propylene glycol, polyethylene glycol, vegetable oils, such as olive oil, and injectable esters, such as ethyl oleate. As a base for the suppository, witepsol, macrogol, Tween 61, cacao butter, laurin paper, glycerolgelatin, etc. may be used.

Specific formulations of pharmaceutical compositions are known in the art and reference can be made to, for example, Remington's Pharmaceutical Sciences (19th ed., 1995). The above reference is considered a part of the present specification.

The pharmaceutical composition of the present disclosure may be administered to mammals such as mouse, dog, cat, cow, horse, pig, a human, and preferably a human, via a variety of routes. Any mode of administration can be envisaged and may include, for example, but is not limited to, oral, intravenous, intraarterial, intramuscular, or subcutaneous injection.

As an example, the oncolytic virus of the present disclosure is resistant to the human complement system and not only maintains stable oncolytic activity upon intravenous injection, but also maximizes therapeutic efficacy by reducing the dosage of oncolytic virus, so that sufficient therapeutic efficacy may be exhibited even with intravenous administration.

In another aspect to achieve the above objectives, the present disclosure provides a genetic construct for insertion into an oncolytic virus, comprising all or part of a gene encoding CD55 and CD59 and operably linked to a promoter for expression, and a vector containing the construct.

In the present disclosure, the gene construct may be for insertion into an inactivated thymidine kinase gene region of an oncolytic virus.

In the present disclosure, the inactivated thymidine kinase gene region includes the deletion of all or part of the thymidine kinase gene.

In the present disclosure, the genetic construct includes one that is located between a J1R region and a J3R region in an oncolytic virus.

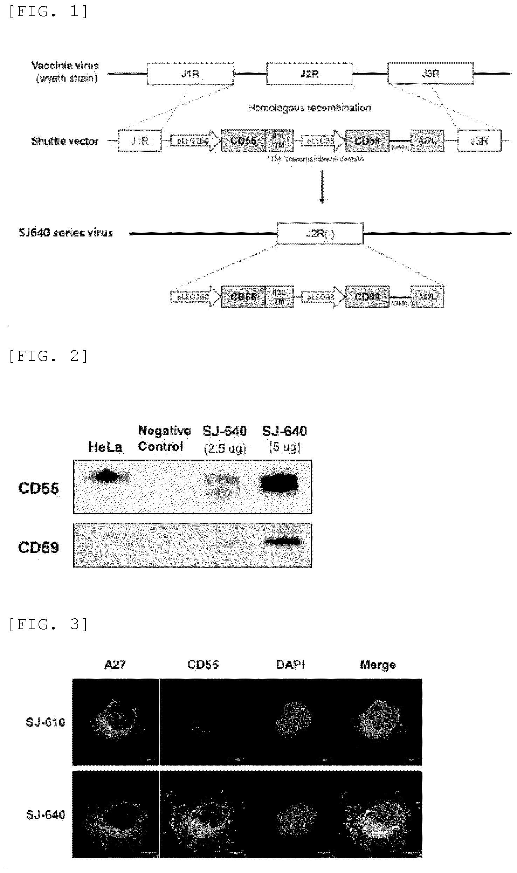

In the present disclosure, the gene construct may include, but is not limited to, those exemplified in FIG. 1 and may further include various promoters and control sequences to regulate expression of the gene in an operably linked form.

As used herein the terms "transmembrane domain," "CD55," and "CD59" are as defined above.

In the present disclosure, the genetic construct is for insertion into all or part of the thymidine kinase of the vaccinia virus, and specifically, for insertion into all or part of a J2R region of the thymidine kinase.

As another aspect for achieving the above objectives, the present disclosure provides an anticancer adjuvant comprising the recombinant vaccinia virus as an active ingredient.

The anticancer adjuvant refers to any form for enhancing the anticancer effect of an anticancer agent or suppressing or ameliorating the side effects of an anticancer agent. The anticancer adjuvant of the present disclosure may be administered in combination with various types of anticancer agents or anticancer adjuvants, and even if the anticancer agent is administered at a lower dose than the conventional anticancer agent when administered in combination, the same level of anticancer treatment effect may be exhibited, such that safer anticancer treatment may be performed.

The anticancer adjuvant may be administered via any common route as long as it can reach the target tissue. The anticancer adjuvant of the present disclosure may be administered intraperitoneally, intravenously, intramuscularly, subcutaneously, intrapulmonary, or intrarectally, depending on the intended use, but is not limited thereto. Furthermore, the anticancer adjuvant may be administered via any device capable of transporting an active substance to a target cell.

The anticancer adjuvant of the present disclosure may be preferably formulated as an anticancer adjuvant by additionally including one or more pharmaceutically acceptable carriers in addition to the active ingredient for administration. Examples of carriers, excipients, or diluents that may be included in the anticancer therapeutic adjuvants of the present disclosure include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils.

The anticancer adjuvant of the present disclosure may be a preparation for parenteral administration, and the description of the preparation is replaced by the description of the preparation of the pharmaceutical composition.

In addition, any anticancer adjuvant disclosed in the art is applicable to the present disclosure without significant limitation.

In one embodiment of the present disclosure, the anticancer agent may be one or more selected from, but not limited to, chemotherapeutic agents, biological therapeutic agents, radiotherapy agents, immunotherapy agents, hormonal therapeutic agents, anti-vascular therapeutic agents, cryotherapy agents, and toxin therapeutic agents.

In another aspect to achieve the above objectives, the present disclosure provides a method of preventing or treating cancer comprising administering to a subject a composition comprising the recombinant vaccinia virus as an active ingredient.

As used herein, the terms "cancer," "prevention," and "treatment" are as defined above.

The individual may be a mammal, specifically, but not limited to, a human, cow, sheep, goat, horse, pig, dog, cat, rabbit, rat, mouse, fish, bird, etc.

The composition comprising the recombinant vaccinia virus as an active ingredient may be appropriately administered by those skilled in the art, depending on the patient's age, sex, weight, disease severity, and administration route. The administration may be once daily or several times daily, and may be repeated at appropriate intervals.

The dosage of the composition comprising the recombinant vaccinia virus as an active ingredient depends on the condition and weight of the individual, the disease severity, the drug form, the route and duration of administration, and may be appropriately selected by those skilled in the art. Specifically, it may be administered to the individual at 10⁵ to about 10¹³ plaque forming units (pfu), and may include various values or ranges between the above ranges.

In the methods of treating cancer of the present disclosure, the composition may be administered via any general route of administration as long as it can reach the target tissue. The composition of the present disclosure may be administered by, but is not particularly limited to, oral administration, intrarectal administration, etc., and in some cases, by other routes depending on the purpose.

In the present disclosure, the method of treating cancer includes a method of treating through combined administration with conventionally known anticancer agents and anticancer therapies.

Another aspect of the present disclosure to achieve the above objectives provides a use of the oncolytic virus or a composition comprising oncolytic virus, for the prevention or treatment of cancer.

Another aspect of the present disclosure to achieve the above objectives provides for a use of the oncolytic virus or a composition comprising the oncolytic virus, for the manufacture of a medicament for the prevention or treatment of cancer.

### [Advantageous Effects]

The oncolytic virus of the present disclosure maintains efficacy even upon intravenous injection and thus can be applied to the therapy for various solid carcinomas and metastatic cancers as well as superficial solid tumors. In addition, by expressing a complement regulatory protein, the oncolytic virus of the present disclosure has resistance from the human complement system and maintains stable oncolytic activity especially upon intravenous injection. Thus, the use of the virus at a reduced dose can minimize adverse effects of anticancer agents. Therefore, the oncolytic virus of the present disclosure can be advantageously used for prevention or treatment of cancer.

### [Description of Drawings]

FIG. 1 shows a schematic diagram of a recombinant vaccinia virus, an oncolytic virus of the present disclosure, in which a TK gene has been deleted and complement regulatory proteins CD55 and CD59 have been introduced.
FIG. 2 shows the result of a Western blot analysis confirming whether a recombinant vaccinia virus (SJ-640) of the present disclosure expresses CD55 and CD59.
FIG. 3 shows the result of immunofluorescence staining confirming whether CD55 is expressed in osteosarcoma cells infected with the recombinant vaccinia virus (SJ-640) of the present disclosure.
FIG. 4 shows the result of immunofluorescence staining confirming whether CD59 is expressed in osteosarcoma cells infected with the recombinant vaccinia virus (SJ-640) of the present disclosure.
FIG. 5 shows the result of confirming the expression of CD55 and CD59 in the recombinant vaccinia virus (SJ-640) of the present disclosure using a transmission electron microscopy.
FIG. 6 shows the result of confirming the serum stability of the recombinant vaccinia virus (SJ-640) of the present disclosure in 20% active human serum.
FIG. 7 shows the result of confirming oncolytic efficacy of SJ-640 in an MDA-MB-231 breast cancer xenograft model through a change in tumor volume.
FIG. 8 shows the result of confirming a change in body weight over time after administration of SJ-610 and SJ-640 to the MDA-MB-231 breast cancer xenograft model.

### [Best Mode]

Hereinafter, the present disclosure will be described in more detail with reference to the following Examples. However, these Examples are intended to illustrate the present disclosure by way of example, and the scope of the present disclosure is not limited to these Examples.

The present inventors produced a vector into which human CD55 and human CD59 genes were inserted, and generated a recombinant vaccinia virus in which the expression of the genes was induced by homologous recombination between the vector and the vaccinia virus, and confirmed its properties as an anticancer agent.

### Preparation Example: Preparation of TK-deleted recombinant vaccinia virus SJ-640 expressing CD55 and CD59

As shown in FIG. 1, the J2R region encoding the thymidine kinase was completely deleted in the Wyeth strain of the vaccinia virus, and CD55 and CD59 were inserted at this site. In addition, in order to express the CD55 on the viral outer membrane, the gene encoding hCD55, without the GPI anchor, was fused with the transmembrane domain of the vaccinia virus membrane protein, and pLEO160 was used as a promoter for expression. In addition, in order to express the CD59 on the viral outer membrane, the gene encoding hCD59, without the GPI anchor, was linked to the vaccinia virus membrane protein using a linker, and pLE038 was used as a promoter for expression. The specific process is as follows.

### 1. Construction of plasmid vectors inducing expression of hCD55 and hCD59 genes

In order to delete a J2R region of vaccinia virus, J1R and J3R genes, which are the left and right flanking regions of the J2R region, were cloned into a T-blunt^{™} vector (Solgent) using a NEBuilder^{®} HiFi DNA Assembly Cloning Kit (NEW ENGLAND BioLabs, Catalog No. E2621). J1R and J3R DNA were generated by PCR amplifying the corresponding positions of JX-594 (Pexastimogene Devacirepvec, Pexa-Vec). The J1R is identical to the region from amino acids 1 to 154 of J1R (Protein id = AAR17936.1) of vaccinia virus Acambis2000 strain, but amino acid position 118 among them is substituted from alanine to serine. J3R (Protein id = AAR17938.1) is the region from amino acids 1 to 145. Then a complement regulatory protein hCD55 gene (GenBank: NM_000574.3) expressed by a pLEO160 promoter, a transmembrane domain region of a vaccinia virus membrane protein H3L, a complement regulatory protein hCD59 gene (Genbank: NM_203331.3) expressed by a pLE038 promoter, a (G4S)₃ linker (SEQ ID NO: 8) linking the hCD59 to the vaccinia virus membrane protein, and a gene encoding a vaccinia virus membrane protein A27L, were cloned into this vector to construct a shuttle vector. The amino acid sequence and nucleotide sequence of the hCD55 used are shown in SEQ ID NOs: 1 and 2, respectively, and the amino acid sequence and nucleotide sequence of the hCD59 are shown in SEQ ID NOs: 3 and 4, respectively.

hCD55 was amplified by PCR from pCMV3 plasmid vector (Sinobio, Catalogue No. HG10101-UT). For hCD55 (GenBank: NM_000574.3), the region corresponding to amino acid 1 to 352, excluding the GPI anchor region, was used from the entire amino acid sequence, and the promoter for expression was pLEO160 (ttttattttttttttttttttggaatataaatatccggtaaaattgaaaaaaaatata cactaattagcgtctcgtttcagacgctagccggtaccccgggttcgaaatcgataagc ttggatccggagagctcccaacctcgaggaattcggtaccccgggttcgaaatcgataa gcttggatccggagagctcccaacctcgagctagctcgag, SEQ ID NO: 5, commissioned for synthesis by Macrogen). The 3'-end of the hCD55 gene was linked to the transmembrane domain region of vaccinia virus H3L to express CD55 on the viral outer membrane, and information and nucleotide sequences of the transmembrane domain region of H3L used are shown in Tables 2 and 3.

**[Table 2]**

| Virus | Membrane protein | Transmembrane domain region | | |
|---|---|---|---|---|
| | | Position | transmembrane | intravirion |
| SJ-640 | H3L | 279-318 | 279-299 | 300-318 |

**[Table 3]**

| Membrane protein | | Sequence | SEQ ID NO: |
|---|---|---|---|
| H3L | Amino acid | LFDINVIGLIVILFIMFMLIFNVKSKLLWFL TGTFVTAFI | SEQ ID NO: 9 |
| | DNA | ttgtttgatattaatgttataggtttgattgtaattttgtttattatgtttat gctcatctttaacgttaaatctaagctgttatggttccttacaggaa cattcgttaccgcatttatc | SEQ ID NO: 10 |

hCD59 was amplified by PCR from cDNA of human cervical cancer cell HeLa. For hCD59 (GenBank: NM_203331.3), the region corresponding to amino acid 1 to 101, excluding the GPI anchor region, was used from the entire amino acid sequence, and the promoter for expression was pLEO38 (ttttattttttttttttttttggaatataaatatccggtaaaattgaaaaaatataca ctaattagcgtctcgtttcagacgctagctcgag, SEQ ID NO: 6, commissioned for synthesis by Macrogen). The 3'-end of the hCD59 gene was linked to a gene encoding vaccinia virus A27L through the linker (G4S)₃ to express CD59 on the viral outer membrane, and the sequences of the A27L and linker used are shown in Table 4.

**[Table 4]**

| Section | | Sequence | SEQ ID NO: |
|---|---|---|---|
| A27L | Amino acid | MDGTLFPGDDDLAIPATEFFSTKAAKKPE AKREAIVKADEDDNEETLKQRLTNLEKK ITNVTTKFEQIEKCCKRNDEVLFRLENHA ETLRAAMIS LAKKIDVQTGRRPYE | SEQ ID NO: 11 |
| | DNA | atggacggaactcttttccccggagatgacgatcttgcaattcca gcaactgaatttttttctacaaaggctgctaaaaagccagaggct aaacgcgaagcaattgttaaagccg atgaagacgacaatgaggaaactctcaaacaacggctaactaat ttggaaaaaaagattactaatgtaacaacaaagtttgaacaaata gaaaagtgttgtaaacgcaacga tgaagttctatttaggttggaaaatcacgctgaaactctaagagcg gctatgatatctctggctaaaaagattgatgttcagactggacgg cgtccatatgag | SEQ ID NO: 12 |
| (G4S)₃ | Amino acid | GGGGSGGGGSGGGGS | SEQ ID NO:7 |
| | DNA | ggtggtggcggttcaggcggaggtggctctggcggtggcgga tcg | SEQ ID NO:8 |

### 2. Preparation of recombinant vaccinia virus SJ-640 by homologous recombination

The recombinant vaccinia virus SJ-640 was prepared by homologous recombination of the wild-type Wyeth strain of vaccinia virus and the previously constructed plasmid vector in which the expression of hCD55 and hCD59 genes was induced.

The wild-type Wyeth strain was prepared by inserting J2R gene of vaccinia virus Western Reserve (WR) strain (ATCC, Catalogue No. VR-1354) into an inactivated J2R region (TK region) of JX-594 (Pexastimogene Devacirepvec, Pexa-Vec). The J2R of Western Reserve strain (Protein id = YP_232976.1) was the region from amino acids 1 to 178. The J2R gene of the Western Reserve strain was amplified by PCR, and the wild-type Wyeth strain was generated by homologous recombination of the PCR product and JX-594.

In order to construct SJ-640, 143B osteosarcoma cells (Creative Bioarray) were infected with the wild-type Wyeth strain described above, and transfected with plasmid vectors expressing hCD55 and hCD59. In order to select the recombinant virus SJ-640 plaques in which the TK region was deleted, the cell lysate in which 143B osteosarcoma cells were infected with vaccinia virus and transfected with the plasmid vector was treated with the TK deletion selection reagent 5-bromo-2'-deoxyuridine (BrdU), and cultured. The selected recombinant virus plaques were further subcultured twice in 143B cells again to obtain purified single clone SJ-640. The nucleotide sequence of the recombination site of SJ-640 was finally confirmed through sequencing.

### Example 1: Confirmation of CD55 and CD59 expression of SJ-640

The recombinant vaccinia virus SJ-640 prepared in the above Preparative Example was lysed, and protein expression was confirmed by Western blotting technique. A virus (JX-594) that does not express CD55 and CD59 was used as a control.

Specifically, HeLa cells were infected with the recombinant vaccinia virus SJ-640 for approximately 26 hours to proliferate, and the infected cells were centrifuged to remove the culture medium, and then cell lysis buffer was added to lyse the cells. The cell lysate was treated with Benzonase to remove cell-derived DNA, and filtered through a cellulose acetate filter to remove cell by-products. The filtrate was concentrated using a 36% sucrose cushion centrifugation method.

In order to isolate proteins present in the purified virus, the centrifuged virus precipitate was lysed with RIPA lysis buffer (Thermo scientific, Catalog No. 89900), and the amount of protein was quantified by BCA protein assay (Thermo scientific, Catalog No. 23227). Proteins obtained from each virus were diluted in loading buffer (Biosesang, Catalog No. S2002) and prepared to be loaded at 2.5 ug or 5 ug per lane, and electrophoresis was performed on SDS-PAGE gel (BIO-RAD, Catalog No. 4561083). The electrophoresed gel was then transferred to a nitrocellulose membrane (BIO-RAD, Catalog No. 1704270). To detect the desired protein, membrane was blocked with 5% skim milk (Difco, Catalog No. 232100) for 1 hour, and treated with an anti-hCD55 antibody (SantaCruz, Catalog No. sc-51733) and an anti-hCD59 antibody (SantaCruz, Catalog No. sc-133171) overnight at 4°C. The next day, the membrane was washed 3-4 times with 1X TBST, treated with the secondary antibody conjugated with HRP, and then treated with hydrogen peroxide and luminol substrate, and chemiluminescence was confirmed with a detector.

As a result, as shown in FIG. 2, it was confirmed that CD55 (about 70 to 75 kDa) and CD59 (about 35 kDa) were expressed on SJ-640.

In addition, osteosarcoma cells U-2 OS were infected with the recombinant vaccinia virus SJ-640 prepared in the above Preparation Example, and after 10 hours, it was confirmed by immunofluorescence staining whether CD55 and CD59 were expressed, respectively. TK-deleted SJ-610 that does not express CD55 and CD59 was used as a negative control.

Specifically, U-2 OS was infected with the recombinant vaccinia virus SJ-640 or the negative control SJ-610, and at 10 hours after infection, virus-infected cells were fixed with 4% paraformaldehyde. The fixed infected cells were then treated with 0.1% Triton X-100 to permeabilize the cell membrane, and blocked by treatment with 3% bovine serum albumin. For immunofluorescence staining of the desired protein, they were treated with an anti-hCD55 antibody (Invitrogen, Catalog No. MA1-82066), an anti-hCD59 antibody (Invitrogen, Catalog No. MA1-19133), and an anti-vaccinia virus A27 antibody (abcam, Catalog No. ab35219) overnight at 4°C. The next day, the primary antibody was washed with PBS, and treated with a secondary antibody conjugated with AlexaFluor488 or AlexaFluor594 that binds to each primary antibody and 4'6-diamidino-2-phenylindole (DAPI), and the expression of vaccinia virus A27 protein, CD55, CD59 and DAPI in infected cells was observed with a confocal microscope.

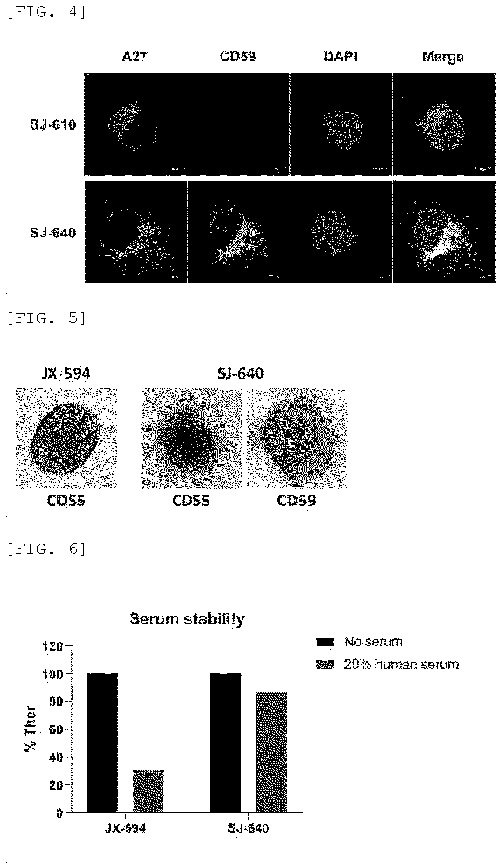

As a result, as shown in FIG. 3, it was confirmed that CD55 was expressed in cells infected with the virus SJ-640, and as shown in Fig. 4, it was confirmed that CD59 was expressed in cells infected with the virus SJ-640. Expression of CD55 and CD59 was not observed in SJ-610 used as a control.

In addition, CD55 or CD59-specific antibodies were bound to the recombinant vaccinia virus SJ-640 prepared in the above Preparation Example, and then immunogold labeled, and the presence, expression sites, and distribution of CD55 and CD59 were observed.

To this end, HeLa cells were infected with the recombinant vaccinia virus SJ-640 for approximately 28-30 hours to proliferate, and the infected cells were centrifuged to remove the culture medium, and then cell lysis buffer was added to lyse the cells. The cell lysate was treated with Benzonase to remove cell-derived DNA, and filtered through a cellulose acetate filter to remove cell by-products. The filtrate was concentrated using a 36% sucrose cushion centrifugation method. The concentrated virus was passed through an ion exchange chromatography membrane to adsorb only the virus and remove impurities, and then the virus was eluted and finally purified.

The purified virus was adsorbed on a nickel grid, and blocked with 3% bovine serum albumin. An anti-hCD55 antibody (SantaCruz, Catalog No. sc-51733) or anti-hCD59 antibody (Invitrogen, Catalog No. MA1-19133) that binds to the desired protein was reacted at room temperature for 2 hours, washed with PBS, and reacted with a secondary antibody (Jacksonimmuno, Catalog No. 115-205-166) conjugated with 12 nm colloidal gold particles at room temperature for 2 hours. Samples prepared for transmission electron microscopy were negatively stained, and the expression of gold-labeled CD55 and CD59 was evaluated.

As a result, it was confirmed that CD55 and CD59 were expressed in SJ-640, and it was confirmed that they were distributed in large numbers on the outer membrane of mature virions (MVs) (Fig. 5). Expression of CD55 and CD59 was not observed in JX-594, which was used as a control virus.

### Example 2: Confirmation of virus stability in serum of SJ-640

In order to confirm whether the SJ-640 virus maintains its activity well in serum, its stability in serum was measured.

Specifically, U-2 OS osteosarcoma cells were seeded in a 12-well tissue culture plate at 2.5 x 10⁵ cells per well and cultured overnight. The next day, when the cells reached almost 100% confluency, the recombinant vaccinia virus SJ-640 was diluted to approximately 80-100 PFU per well in serum-free DMEM medium. Normal human serum was then added to the diluted virus to 20% of the total volume, and treated for 2 hours in the plate in which the U-2 OS cells were cultured. As a control group, serum-free DMEM medium was used instead of the normal human serum. After 2 hours, the virus was removed from the 12-well plate and treated with 1.5% carboxymethyl cellulose solution. The 12-well plate was cultured at 37°C for 72 hours, and plaques formed were stained with 0.1% crystal violet solution and counted. When the number of plaques generated in a case where serum was not included was 100%, the number of plaques generated from the recombinant virus treated with 20% normal human serum and cultured was calculated, and the stability in serum of the recombinant virus SJ-640 was compared with that of the negative control JX-594.

As a result, as shown in FIG. 6, it was confirmed that the activity of the control JX-549 was reduced to 30%, while the activity of the virus SJ-640 of the present invention was maintained up to 87%.

### Example 3: Evaluation of anti-tumor efficacy in a breast cancer transplant model

A xenograft mouse tumor model was established by transplanting the human breast cancer cells MDA-MB-231 into NSG immunodeficient mice. When the tumor volume reached approximately 120 mm³, 1 x 10⁶ pfu of SJ-640 or the control SJ-610 was administered intravenously as a single dose, and the tumor growth inhibitory efficacies were compared.

Tumor volumes were measured in the long axis and short axis using a caliper 2-3 times a week in all groups. Tumor volume was calculated as (short axis x short axis x long axis) ÷ 2. In addition, in order to evaluate the body weight of the mouse, the weight was measured at each time point of measuring the tumor volume. The experiment was terminated when the tumor volume reached 1,500 to 2,000 mm³ or more.

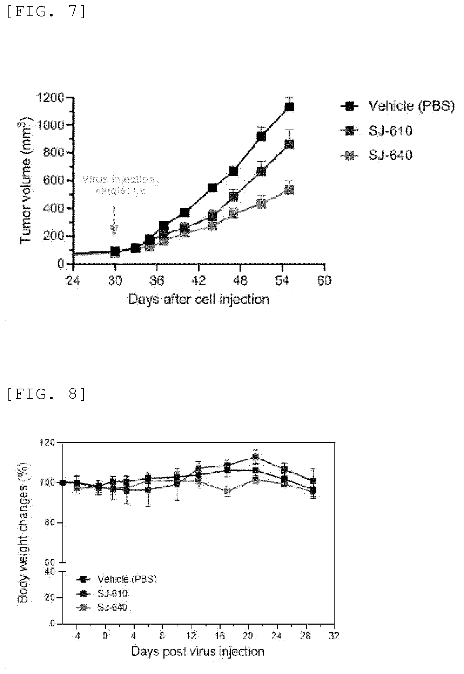

As a result, as shown in FIG. 7, it was confirmed that the recombinant vaccinia virus SJ-640 of the present invention showed a tumor growth inhibitory efficacy of 52.9% compared to the untreated control group (PBS), and the tumor growth inhibitory efficacy of SJ-610 was 23.6%, and thus, the recombinant vaccinia virus SJ-640 of the present invention had a remarkably excellent anti-tumor effect.

In addition, as shown in Fig. 8, there was no statistically significant difference in the change in body weight of mice in all experimental groups, and no clinical toxicity was observed.

From the above description, those skilled in the art to which the present disclosure pertains will understand that the present disclosure can be implemented in other specific forms without changing its technical idea or essential features. In this regard, it should be understood that the embodiments described above are exemplary in all respects and are not intended to be limiting. The scope of the present disclosure should be interpreted that all changes or modifications derived from the meaning and scope of patent claims to be described below rather than the detailed description above and their equivalent concepts are included in the scope of the present disclosure.

## Claims

1. An oncolytic virus co-expressing CD55 and CD59.

2. The oncolytic virus of claim 1, wherein the oncolytic virus has a suppressed expression of a thymidine kinase gene.

3. The oncolytic virus of claim 2, wherein the suppressed expression of the thymidine kinase gene is due to deletion of part or all of the gene, or insertion of a foreign gene into the gene.

4. The oncolytic virus of claim 2, wherein the foreign gene is inserted into part or all of the thymidine kinase J2R region.

5. The oncolytic virus of claim 1, wherein the CD55 is composed of an amino acid sequence of SEQ ID NO: 1.

6. The oncolytic virus of claim 1, wherein the CD59 is composed of an amino acid sequence of SEQ ID NO: 3.

7. The oncolytic virus of claim 1, further comprising a gene encoding an oncolytic virus membrane protein or a transmembrane domain thereof.

8. The oncolytic virus of claim 7, wherein the gene encoding the oncolytic virus membrane protein is linked to a gene encoding the CD59 through a linker.

9. The oncolytic virus of claim 8, wherein the linker is composed of an amino acid sequence of SEQ ID NO: 7.

10. The oncolytic virus of claim 7, wherein the gene encoding the transmembrane domain of the oncolytic virus membrane protein is fused with a gene encoding the CD55.

11. The oncolytic virus of claim 7, wherein the membrane protein of the oncolytic virus is H3L, A27L, D8L, A16L, F9L, G9R, L1R, A9L, A13L, A21L, A28L, E10R, G3L, H2R, I2L, J5L, L5R, or O3L.

12. The oncolytic virus of claim 1, wherein the CD55 and CD59 are expressed under the control of a late-early VACV p7.5 promoter, a vaccinia synthetic early-late promoter (pSEL), a vaccinia synthetic late promoter (pSL), a vaccinia modified H5 (mH5) promoter, a vaccinia short synthetic early-late pS promoter, a pLEO160 promoter, a pLEO38 promoter, a pLate promoter, a pC11R promoter, a pF11L promoter, a psFJ1-10 synthetic early promoter, a pHyb synthetic early promoter, any natural vaccinia early promoter, or a late-early optimized (LEO) promoter, respectively.

13. The oncolytic virus of claim 1, wherein the oncolytic virus is a vaccinia virus, an adenovirus, a herpes simplex virus, a retrovirus, a reovirus, a Newcastle disease virus, a Coxsackie virus, an enterovirus, or a herpes virus.

14. The oncolytic virus of claim 13, wherein the vaccinia virus is Western Reserve (WR), New York Vaccinia Virus (NYVAC), Wyeth, LC16m8, Lister, Copenhagen, Tian Tan, USSR, TashKent, Evans, International Health Division-J (IHD-J), or International Health Division-White (IHD-W) strain.

15. A pharmaceutical composition for preventing or treating cancer, comprising the oncolytic virus of any one of claims 1 to 14 as an active ingredient.

16. The pharmaceutical composition of claim 15, wherein the cancer is solid cancer or haematological cancer.

17. The pharmaceutical composition of claim 16, wherein the solid cancer is any one selected from the group consisting of lung cancer, colorectal cancer, prostate cancer, thyroid cancer, breast cancer, brain cancer, head and neck cancer, esophageal cancer, skin cancer, thymic cancer, stomach cancer, colon cancer, liver cancer, ovarian cancer, uterine cancer, bladder cancer, rectal cancer, gallbladder cancer, bile duct cancer, pancreatic cancer, kidney cancer, osteosarcoma, sarcoma, chondrosarcoma, and combinations thereof.

18. The pharmaceutical composition of claim 16, wherein the haematological cancer is any one selected from the group consisting of lymphoma, leukaemia, multiple myeloma, and combinations thereof.

19. The pharmaceutical composition of claim 15, wherein the pharmaceutical compositions is for intratumoral, intravascular, intramuscular, or intraperitoneal administration.

20. The pharmaceutical composition of claim 15, wherein the pharmaceutical compositions is for intravenous or intraarterial administration.

21. A gene construct for insertion into an oncolytic virus, comprising all or part of a gene encoding CD55 and CD59, and operably linked to a promoter for expression.

22. The gene construct of claim 21, wherein the gene construct is for insertion into an inactivated thymidine kinase gene region of the oncolytic virus.

23. An anticancer adjuvant comprising the oncolytic virus of any one of claims 1 to 14 as an active ingredient.

24. A method of preventing or treating cancer, comprising administering to a subject a composition comprising the oncolytic virus of any one of claims 1 to 14 as an active ingredient.

25. Use of the oncolytic virus of any one of claims 1 to 14 or a composition comprising the oncolytic virus, for the prevention or treatment of cancer.

26. Use of the oncolytic virus of any one of claims 1 to 14 or a composition comprising the oncolytic virus, for the manufacture of a medicament for the prevention or treatment of cancer.
